# EUROPEAN PATENT APPLICATION

(11) **EP 1 547 516 A1**
(43) Date of publication of application: **29.06.2005**
(21) Application number: 03741375.4
(22) Date of filing: 14.07.2003
(51) Int. Cl.: A61B 5/00, G06F 17/60, A61M 1/14

(54) **MEDICAL DATA WARNING NOTIFYING SYSTEM AND METHOD**

(30) Priority: 15.07.2002 JP 2002205871
(71) Applicant: JMS Co., Ltd., Hiroshima-shi, Hiroshima 730-8652 (JP)
(72) Inventor: KAWAMOTO, Atsushi, Hiroshima-shi, Hiroshima 731-0231 (JP); NAKAMOTO, Hidetomo, Nerima-ku, Tokyo 179-0084 (JP); NISHIDA, Eiichi, Kitakyushu-shi, Fukuoka 805-0016 (JP)
(74) Representative: Power, Philippa Louise
(86) International application number: PCT/JP2003/008890
(87) International publication number: WO 2004/006758

(57) **Abstract**

A medical data warning notifying system and method for notifying a hospital only when there is abnormal data in medical data entered by a patient on peritoneal dialysis at home. In the medical data warning notifying system, a patient terminal used by a patient, a server for controlling data exchanged between the patient and a hospital, and a hospital terminal used by a doctor are interconnected over a network. The server receives medical data entered through the patient terminal, stores the received medical data, and judges from the received medical data whether or not there is any abnormal data. If it is judged that abnormal data is included, a warning signal is sent to the hospital terminal. A message created on the hospital terminal is relayed and sent to the patient terminal.

## Description

### TECHNICAL FIELD

The present invention relates to a medical data warning notifying system and method for detecting abnormalities of the measured data when a patient uses a medical measurement device at home.

### BACKGROUND ART

When performing peritoneal dialysis, the interior of the peritoneum is filled with a dialysate having a concentration gradient with respect to the bodily fluid of a patient by a catheter that leads to the peritoneal cavity through the abdominal wall of the patient. The peritoneum of the patient functions as a semi-permeable membrane and excess water, toxin, waste products, and the like inside the body of the patient move into the peritoneal cavity. Then the excess water, toxin, waste products, and the like inside the peritoneal cavity are drained to an exterior of the body along with an effluent.

More specifically, through an extension tube or the like connected to a dialysate bag, a drainage bag, and the catheter, the dialysate is infused into the peritoneal cavity. After the infusion, the solution is retained for a predetermined time (3 to 5 hours), and the solution in which the toxin, waste products, and the like have accumulated is drained to the drainage bag. Thus the dialysate is infused into the peritoneal cavity of the patient through the tube and the solution of the toxin, waste products, and the like is also drained to the drainage bag through the tube.

When it is necessary to perform the dialysis at home and not in a hospital due to the particular circumstances or social environment of the patient, the dialysis is performed based on a dialysis schedule based on a diagnosis at the hospital by a doctor. Measurement data such as an amount of water drained and a change in body weight during the dialysis need to be monitored by the hospital as medical data so that the measurement data can be used in the subsequent diagnosis.

Conventionally, in such a situation, CAPD patients only input and send the measurement data such as the amount of effluent solution, the amount of water drained, body weight, blood pressure, and the like of each run of the dialysis to the hospital, and the sent data are evaluated by performing an analysis in the hospital.

However, in the method described above, there is the problem that a considerable amount of time is necessary in confirming and determining whether the state of the patient is normal or abnormal from the moment when the hospital receives the input data, and it is not possible to respond when some form of prescription or the like is needed in an emergency.

In other words, it is practically impossible for the doctors and others at the hospital to confirm and respond to the data that the doctors and others receive one after another, because the data sent to the hospital is so voluminous for each patient. Further, considerable time is also necessary to classify and analyze the data received for each patient. Furthermore, it is not preferable to execute a similar confirmation procedure or analysis process on the normal data in order to detect a few abnormal data, even though almost all data are normal, because doing so puts an unnecessary burden on the medical staff and is a waste of computer resources.

Also, symptoms, body weight and the like of the patient change over time, and needless to say, it is preferable for the patient to change flexibly the projected amount of the water to be drained, the dwell time, or the like according to the circumstances during the dialysis.

In order to solve the problems described above, it is an object of the present invention to provide a medical data warning notifying system and method that is able to notify the hospital only when it is judged that there is abnormal data in the medical data that the patient input in performing the peritoneal dialysis at home.

### DISCLOSURE OF INVENTION

In order to solve the above-described problems, a medical data warning notifying system of the present invention is a medical data warning notifying system in which a patient terminal used by a patient, a server that controls data that is exchanged between the patient server and a hospital server, and the hospital terminal used by a doctor are connected via a network. The server includes a medical data receiving portion for receiving medical data that were input at the patient terminal; a medical data storage portion for storing the received medical data; a judging portion for judging whether or not the received medical data include abnormal data; a warning notifying portion for sending a warning signal to the hospital terminal if it is judged that the medical data include abnormal data; and a message transceiver portion for receiving a message generated by the hospital terminal and for sending the message to the patient terminal.

With the configuration described above, the server determines in advance whether or not there is an abnormality in the medical data that was input by the patient, so that a warning signal can be sent to the hospital only if the medical data includes abnormal data. Thus, not only can the hospital ensure accurate and quick medical data analyses, diagnoses, and the like, but the patient also can expect to receive an appropriate prescription quickly when abnormal data are detected.

Further, in the medical data warning notifying system of the present invention, it is preferable that the judging portion determines that there is an abnormality if given medical data exceed a predetermined threshold value. Alternatively, it is also preferable that received records are stored by type of the medical data, an average value is calculated, and the judging portion determines that there is an abnormality if the difference between the average value and the stored medical data exceeds a predetermined percentage of the average value. In the latter case, it is even more preferable that the received records are records for a predetermined period of time and the average value is an average value for a specified period of time during the predetermined period of time.

Furthermore, in the medical data warning notifying system of the present invention, it is preferable that the warning signal is a signal that changes a display specification of the notified message. Alternatively, it is preferable that the warning signal is a signal that controls a sound output of the hospital terminal receiving the warning signal.

The present invention also relates to software on a server executing the above-described functions of a medical data warning notifying system as process steps on a computer. Specifically, the present invention relates to a medical data warning notifying method provided in an environment in which a patient terminal used by a patient, a server that controls data that is exchanged between the patient terminal and a hospital terminal, and the hospital terminal used by a doctor are connected via a network. The server is operated so as to perform the steps of receiving medical data that were input at the patient terminal; storing the received medical data; judging whether or not the received medical data include abnormal data; sending a warning signal to the hospital terminal if it is judged that the medical data include abnormal data; and receiving a message generated by the hospital terminal and sending the message to the patient terminal; as well as a computer-executable program for implementing such steps.

With the configuration described above, it is possible to realize a medical data warning notifying system in which, by loading such a program and executing it on the computer, the server determines in advance whether or not there is an abnormality in the medical data that were input by the patient, so that a warning signal can be sent to the hospital only if the medical data include abnormal data, and not only can the hospital ensure accurate and quick medical data analyses, diagnoses and the like, but the patient also can expect to receive an appropriate prescription quickly when abnormal data are detected.

### BRIEF DESCRIPTION OF DRAWINGS

FIG. 1 is a block diagram of a medical data warning notifying system of an embodiment of the present invention.
FIG. 2 shows an example of a screen for setting warning notification conditions in a medical data warning notifying system of an embodiment of the present invention.
FIG. 3 shows an example of a warning notification mail in a medical data warning notifying system of an embodiment of the present invention.
FIG. 4 shows an example of a warning notification display in a medical data warning notifying system of an embodiment of the present invention.
FIG. 5 is a flowchart of the process in a medical data warning notifying system of an embodiment of the present invention.
FIG. 6 shows an example of a computer environment.

### BEST MODE FOR CARRYING OUT THE INVENTION

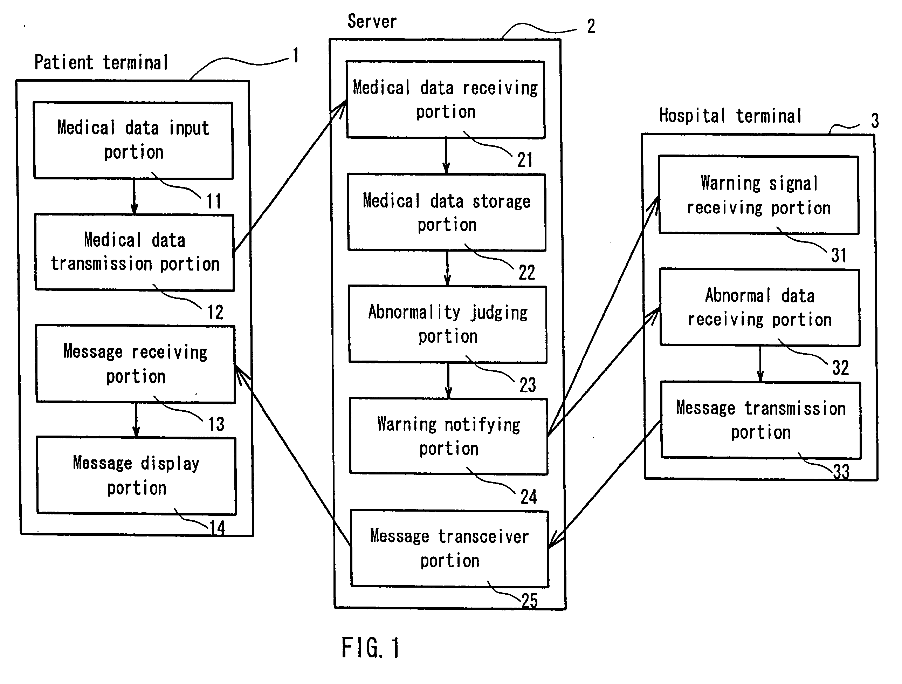

Below, an embodiment of a medical data warning notifying system according to the present invention is explained with reference to the drawings. FIG. 1 is a diagram that illustrates the medical data warning notifying system according to this embodiment of the present invention. In FIG. 1, 1 is a patient terminal that a patient uses, 2 is a server, and 3 is a hospital terminal that a hospital uses.

In FIG. 1, not only a generic personal computer but a portable terminal, or the like as typified by a PDA (personal digital assistant), mobile phone or the like may be used as the patient terminal 1. The patient terminal 1 includes at least a medical data input portion 11 for inputting various kinds of data when the patient performs peritoneal dialysis or the like at home, a medical data transmission portion 12 for sending the input medical data to the server 2, a message receiving portion 13 for receiving messages from the hospital, and a message display portion 14 for displaying the received messages. Here, the "messages" are not limited to text data but include a wide range of concepts from image data that enable a graphical display, sound data or the like as typified by a synthesized sound or the like, or medical data that are processed after the patient's input and converted into display data with which a change in an amount through the course of time, an average value, an abnormal value or the like of, for example, the amount of water drained that can be visualized.

Also, the server 2 controls whether or not the medical data are sent to the hospital terminal 3. The server 2 includes at least a medical data receiving portion 21 for receiving the medical data sent from the patient terminal 1, a medical data storage portion 22 for storing the received medical data, an abnormality judging portion 23 for judging whether or not there is an abnormality in the received medical data, a warning notifying portion 24 for sending warning signals and the corresponding medical data to the hospital terminal 3 when it is judged that there is an abnormality, and a message transceiver portion 25 for receiving the messages from the hospital terminal 3 and sending the messages to the patient terminal 1.

The hospital terminal 3 performs data processing such as performing a data analysis based on the medical data that a doctor examined and sends to the patient terminal 1 the best prescription for the patient, the course of actions that the patient should take, or the like as a message. Therefore, the hospital terminal 3 includes at least a warning signal receiving portion 31 for receiving the warning signals, an abnormal data receiving portion 32 for receiving the medical data that has been judged to be abnormal, and a message transmission portion 33 for sending messages to the patient.

First, the patient inputs various kinds of medical data generated in performing the peritoneal dialysis such as amount of water drained, body weight, maximum blood pressure, minimum blood pressure, pulse rate, and the like from the medical data input portion 11 of the patient terminal 1. The data are sent to the server 2 from the medical data transmission portion 12, received at the medical data receiving portion 21 in the server 2, and stored individually for each patient in the medical data storage portion 22.

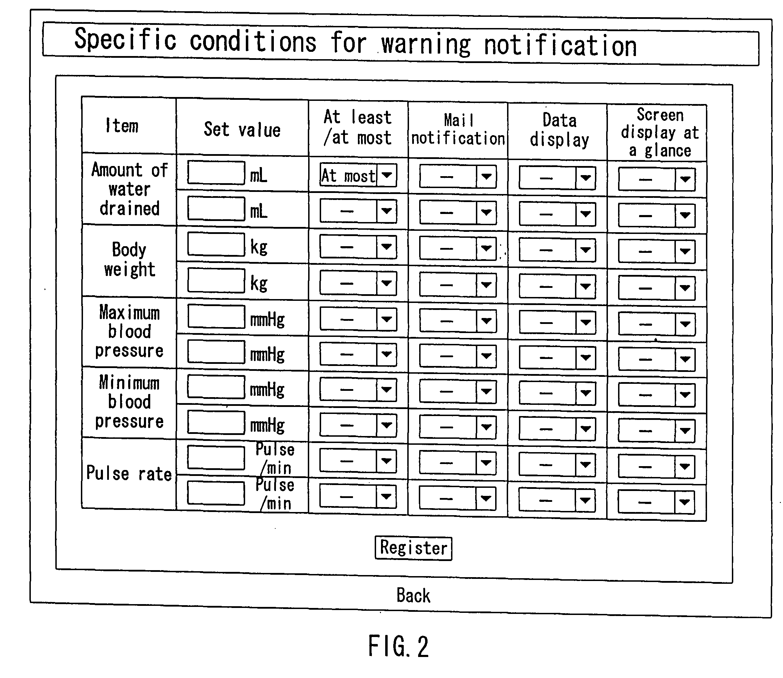

Next, the abnormality judging portion 23 judges whether or not the sent medical data includes abnormal data. Various ways of judgment are possible. The most common method is to set a threshold value for determining whether or not the data are abnormal for each item of the medical data for each patient, and judge by comparing the applicable threshold value and the received medical data. For example, the judgment is made by using a setting screen enabling detailed determination of set values for warning notification, as shown in FIG. 2, inputting the set values for each item of the medical data on the setting screen, and comparing the set values with the received medical data.

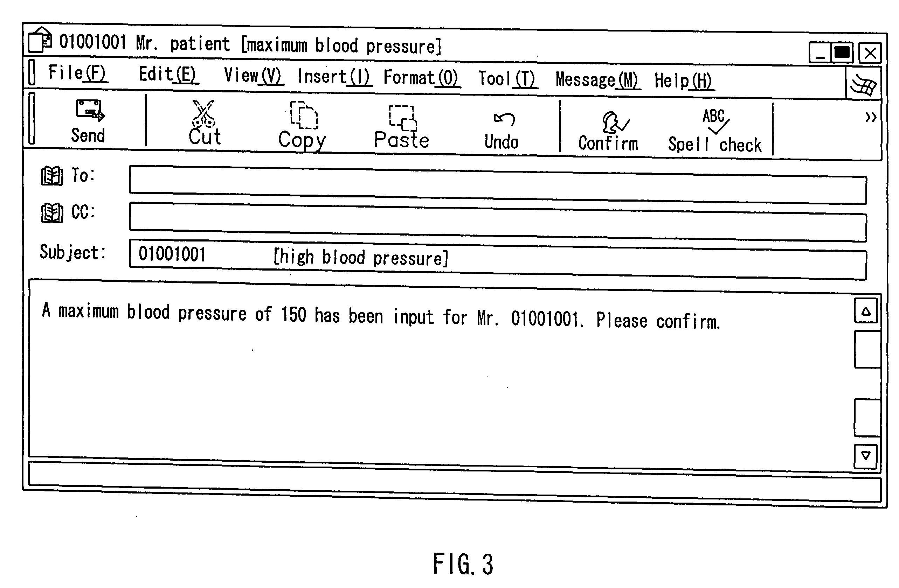

For the "mail notification" in FIG. 2, "ON" or "OFF" can be selected. When "ON" is selected, a message containing an ID identifying the patient, the name of the patient, and the like, along with the abnormal data item, as shown in FIG. 3, is sent to the terminal at the hospital.

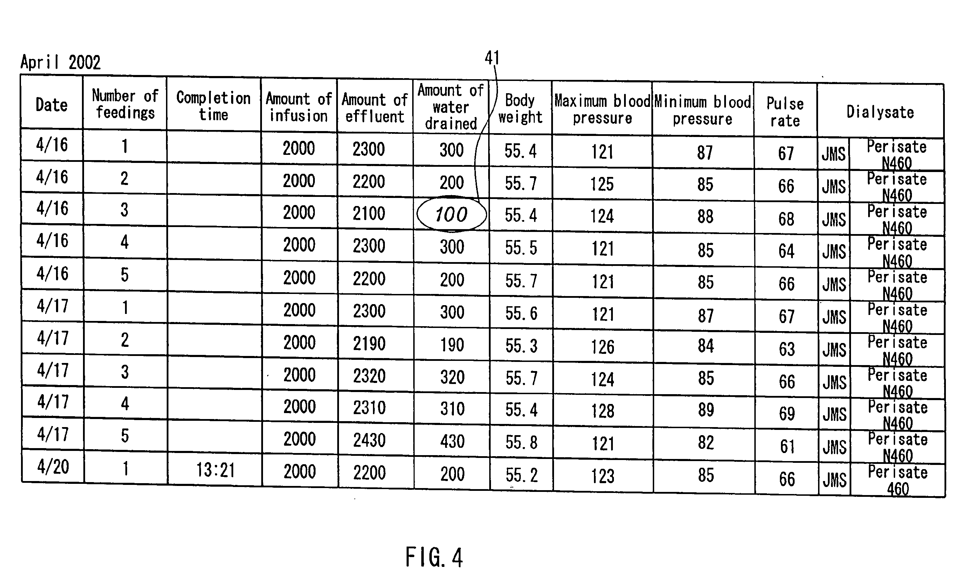

With "data display" in FIG. 2, it is possible to select a color for display such as "red" and "blue." For example, if the amount of water drained is set to "red" with "less than or equal to 150 mL," then only the datum 41 shown in FIG. 4 is displayed in red, thereby allowing the hospital staff to recognize visually that abnormal data are generated.

It is also possible to set a range of values that should be considered as abnormal data or change the method of warning notification according to the circumstances by setting up two set values for each item as shown in FIG. 2. Even if the abnormal data are the same, it is possible to change the method of warning notification according to the severity by setting the mail notification to "ON" if the amount of water drained is "less than or equal to 20 mL" and setting the data display to "red" if the amount of water drained is "less than or equal to 50 mL" for example.

The method of setting the condition for the warning notification is certainly not limited to what is described above and items for which a warning can be given are also not limited to the items shown in FIG. 2.

Furthermore, as a method of judgment, it is also conceivable that whether or not the data are abnormal data are judged by calculating an average value of a predetermined period of time, such as the last 2 to 3 months, based on the past data accumulated in the medical data storage portion 22 for each set of medical data in conjunction with whether or not the difference between the calculated average value and the received medical data are within a predetermined limit. In this case, a judging condition is established, for example by regarding cases in which the absolute value of the difference exceeds 3% of the calculated average value as abnormal data.

If the abnormality judging portion 23 judges that the data are abnormal, then a warning signal and the corresponding medical data are sent to the hospital terminal 3 from the warning notifying portion 24. Other than converting the color of the data display, the warning signals also can be presented by mail or signals causing the output of an audible alarm sound using the sound output function in the hospital terminal 3. The warning signals are not limited to this.

The hospital receives the warning signal and the abnormal data and the best prescription and the like are determined by a doctor. A message concerning the prescription and the like is then generated and sent from the message transmission portion 33 to the patient terminal 1.

In practice, since the message is sent via the server 2, the message transceiver portion 25 in the server 2 receives the message from the hospital terminal 3 and sends the message on to the patient terminal 1. The message receiving portion 13 in the patient terminal 1 receives the message and the message is displayed on the message display portion 14.

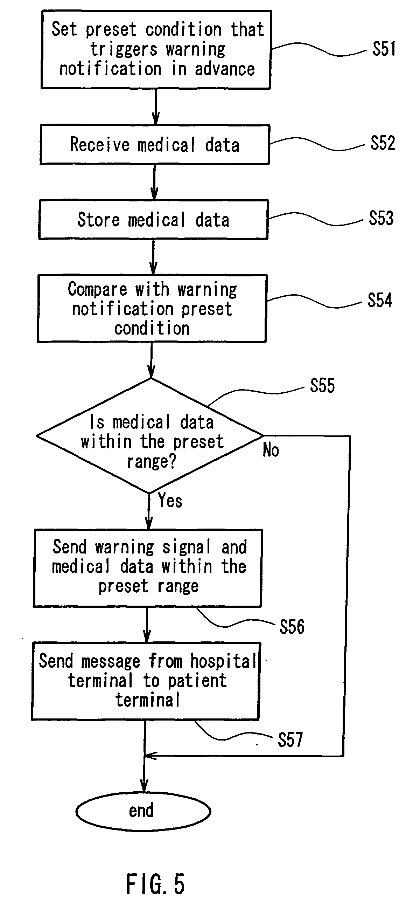

Next, FIG. 5 shows a flowchart of the procedure in the server 2 of the medical data warning notifying system according to this embodiment of the present invention. In FIG. 5, first, the data range at which a warning is to be notified to the hospital terminal 3 is set and registered for each patient in advance (step S51). Next, various kinds of medical data concerning the peritoneal dialysis are received (step S52) and the received data are stored in the medical data storage portion 22 (step S53).

The received medical data then are compared to the preset data range that has been set and registered in advance (step S54) and if the received medical data are within the preset data range (step S55: Yes), then the warning signal and the medical data judged to be within the preset data range, in other words, the medical data judged to be abnormal data, are sent to the hospital terminal 3 (step S56).

Then a standby mode is assumed until a message is received from the hospital terminal 3 and when the message is received, the message is sent to the patient terminal 1 (step S57).

As described above, according to the present embodiment, only when abnormal data are included in the medical data that the patient measured in the peritoneal dialysis performed at home, a warning signal is set, along with the corresponding abnormal data, to the hospital and the appropriate diagnosis result given by the hospital is sent back. Therefore, it is possible to perform a quick and efficient medical procedure as the patient can concentrate on the medical procedure with a sense of safety and the hospital does not waste unnecessary time on a pointless data analysis.

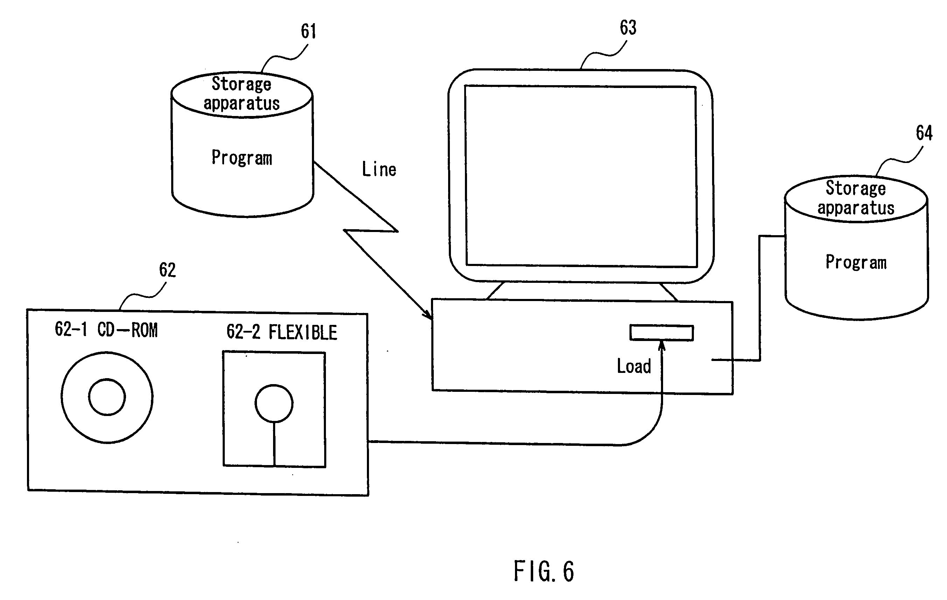

It should be noted a server program implementing the medical data warning notifying system according to this embodiment of the present invention may be recorded not only on a portable recording medium 62 such as a CD-ROM 62-1 or a flexible disc 62-2 as shown in FIG. 6, but also on any other storage apparatus 61 provided on a communication line or a recording medium 64 such as a hard disk or a RAM of a computer 63. When the program is implemented, the program is loaded into a main memory and executed.

Furthermore, the various kinds of medical data and the like that are used by the medical data warning notifying system according to this embodiment of the present invention may be recorded not only on a portable recording medium 62 such as the CD-ROM 62-1 or the flexible disc 62-2 as shown in FIG. 6, but also on any other storage apparatus 61 provided on a communication line or a recording medium 64 such as the hard disk or the RAM of the computer 63. The computer 63 reads the medical data when the medical data warning notifying system according to the present invention is used, for example.

### INDUSTRIAL APPLICABILITY

As described above, according to the medical data warning notifying system according to the present invention, only when abnormal data are included in the medical data that the patient measured in the peritoneal dialysis performed at home, a warning signal is set, along with the corresponding abnormal data, to the hospital and the appropriate diagnosis result given by the hospital is sent back. Therefore, it is possible to perform a quick and efficient medical procedure as the patient can concentrate on the medical procedure with a sense of safety and the hospital does not waste unnecessary time on pointless data confirmation and data analysis.

## Claims

1. A medical data warning notifying system in which a patient terminal used by a patient, a server that controls data that are exchanged between the patient terminal and a hospital terminal, and the hospital terminal used by a doctor are connected via a network,
wherein the server comprises:
a medical data receiving portion for receiving medical data that were input at the patient terminal;
a medical data storage portion for storing the received medical data;
a judging portion for judging whether or not the received medical data include abnormal data;
a warning notifying portion for sending a warning signal to the hospital terminal if it is judged that the medical data include abnormal data; and
a message transceiver portion for receiving a message generated by the hospital terminal and sending the message to the patient terminal.

2. The medical data warning notifying system according to claim 1, wherein the judging portion determines that there is an abnormality if given medical data exceeds a predetermined threshold value.

3. The medical data warning notifying system according to claim 1, wherein received records are stored by type of the medical data, an average value is calculated, and the judging portion determines that there is an abnormality if the difference between the average value and the stored medical data exceeds a predetermined percentage of the average value.

4. The medical data warning notifying system according to claim 3, wherein the received records are records for a predetermined period of time and the average value is an average value for a specified period of time during the predetermined period of time.

5. The medical data warning notifying system according to any one of claims 1 to 4, wherein the warning signal is a signal that changes a display specification of the notified message.

6. The medical data warning notifying system according to any one of claims 1 to 3, wherein the warning signal is a signal that controls a sound output of the hospital terminal receiving the warning signal.

7. A medical data warning notifying method provided in an environment in which a patient terminal used by a patient, a server that controls data that are exchanged between the patient terminal and a hospital terminal, and the hospital terminal used by a doctor are connected via a network,
wherein the server is operated so as to perform the steps of:
receiving medical data that were input at the patient terminal;
storing the received medical data;
judging whether or not the received medical data include abnormal data;
sending a warning signal to the hospital terminal if it is judged that the medical data include abnormal data; and
receiving a message generated by the hospital terminal and sending the message to the patient terminal.

8. A computer-executable program in a server for implementing a medical data warning notifying method provided in an environment in which a patient terminal used by a patient, a server that controls data that are exchanged between the patient terminal and a hospital terminal, and the hospital terminal used by a doctor are connected via a network, the computer executable program comprising the steps of:
receiving medical data that were input at the patient terminal;
storing the received medical data;
judging whether or not the received medical data include abnormal data;
sending a warning signal to the hospital terminal if it is judged that the medical data include abnormal data; and
receiving a message generated by the hospital terminal and sending the message to the patient terminal.
